(19) Europäisches Patentamt

European Patent Office

Office européen des brevets

(11) **EP 1 474 131 B1**

(12) **EUROPEAN PATENT SPECIFICATION**

(45) Date of publication and mention
of the grant of the patent:
**22.11.2006 Bulletin 2006/47**

(21) Application number: **03701674.8**

(22) Date of filing: **13.02.2003**

(51) Int Cl.:
*A61K 31/385* (2006.01)    *A61P 35/00* (2006.01)

(86) International application number:
**PCT/IB2003/000463**

(87) International publication number:
**WO 2003/068219 (21.08.2003 Gazette 2003/34)**

(54) **USE OF ANETHOLE DITHIOLETHIONE IN LUNG CANCER CHEMOPREVENTION**

VERWENDUNG VON ANETHOL-DITHIOLETHION ZUR VORBEUGUNG VON LUNGENKREBS

UTILISATION DE L'ANETHOLE-DITHIOLETHIONE POUR LA CHIMIOPREVENTION DU CANCER DU POUMON

(84) Designated Contracting States:
**AT BE BG CH CY CZ DE DK EE ES FI FR GB GR HU IE IT LI LU MC NL PT SE SI SK TR**

(30) Priority: **13.02.2002 US 355826 P**

(43) Date of publication of application:
**10.11.2004 Bulletin 2004/46**

(73) Proprietor: **Solvay Pharma**
**92150 Suresnes (FR)**

(72) Inventors:
• **LAM, Stephen**
**Vancouver, British Columbia V65 1T8 (CA)**
• **MACAULAY, Calum**
**Vancouver, British Columbia V5W 1E7 (CA)**
• **LE RICHE, Jean**
**Vancouver, British Columbia V6K 253 (CA)**
• **DYACHKOVA, Yulia**
**Vienna 1030 (AT)**
• **COLDMAN, Andy**
**Vancouver, British Columbia (CA)**
• **GUILLAUD, Martial**
**Vancouver, British Columbia V6J 3T6 (CA)**
• **HAWK, Ernest**
**Finksburg, MD 21048 (US)**
• **CHRISTEN, Marie-Odile**
**F-75016 Paris (FR)**
• **GAZDAR, Adi**
**Dallas, TX 75230-5033 (US)**

(74) Representative: **Bernasconi, Jean Raymond et al**
**c/o Cabinet Lavoix,**
**2, Place d'Estienne d'Orves**
**75441 Paris Cédex (FR)**

(56) References cited:
• **REDDY B S ET AL: "Chemoprevention of colon carcinogenesis by organosulfur compounds." CANCER RESEARCH. UNITED STATES 1 AUG 1993, vol. 53, no. 15, 1 August 1993 (1993-08-01), pages 3493-3498, XP001097689 ISSN: 0008-5472**
• **ZHANG L ET AL: "The chemopreventive effect of Sialor (Anethole dithiolethione) on hamster buccal pouch carcinogenesis." PROCEEDINGS OF THE AMERICAN ASSOCIATION FOR CANCER RESEARCH ANNUAL, vol. 39, March 1998 (1998-03), page 641 XP001097690 89th Annual Meeting of the American Association for Cancer Research;New Orleans, Louisiana, USA; March 28-April 1, 1998, March, 1998 ISSN: 0197-016X**
• **LUBET RONALD A ET AL: "Chemopreventive efficacy of anethole trithione, N-acetyl-L-cysteine, miconazole and phenethylisothiocyanate in the DMBA-induced rat mammary cancer model." INTERNATIONAL JOURNAL OF CANCER, vol. 72, no. 1, 1997, pages 95-101, XP002237061 ISSN: 0020-7136**
• **KENSLER T W ET AL: "OLTIPRAZ: CLINICAL OPPORTUNITES FOR CANCER CHEMOPREVENTION" JOURNAL OF CELLULAR BIOCHEMISTRY - SUPPLEMENT, WILEY-LISS, US, vol. 22, 1995, pages 101-107, XP000979453 ISSN: 0730-2312**
• **ANSHER S S DOLAN P BUEDING E: "Chemoprotective effects of two dithiolthiones and of butylhydroxyanisole against carbon tetrachloride and acetaminophen toxicity" HEPATOLOGY, WILLIAMS AND WILKINS, BALTIMORE, MD, US, vol. 3, no. 6, November 1983 (1983-11), pages 932-935, XP002958056 ISSN: 0270-9139**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 474 131 B1

- **LAM STEPHEN ET AL: "A randomized phase IIb trial of anethole dithiolethione in smokers with bronchial dysplasia." JOURNAL OF THE NATIONAL CANCER INSTITUTE (BETHESDA), vol. 94, no. 13, 3 July 2002 (2002-07-03), pages 1001-1009, XP001145846 July 3, 2002 ISSN: 0027-8874**
- **LAM S ET AL: "A phase II clinical trial of anethole dithiolethione (Sialor(R), Sulfarlem(R)) in smokers with bronchial dysplasia" EUROPEAN JOURNAL OF CANCER, PERGAMON PRESS, OXFORD, GB, vol. 38, 14 February 2002 (2002-02-14), page S45 XP004367808 ISSN: 0959-8049**
- **MORSE M.A.: 'Failure of dietary oltipraz to inhibit benzo-pyrene-induced lung tumorigenesis in strain mice' CANCER LETTERS vol. 91, 1995 - 1995, pages 133 - 138**

**Description**

The present invention relates to the use of anethole dithiolethione in lung cancer chemoprevention.

Lung cancer is the most common cause of cancer death worldwide with a mortality rate exceeding that of colon, breast and prostate cancers combined (Greenlee et al., 2001). Former heavy smokers retain an elevated risk for lung cancers even years after they stop smoking (Halpern et al., 1993 ; Tong et al., 1996). With a large reservoir of current and former smokers and the increasing incidence of lung cancers among women, lung cancer will remain a major health issue for the next several decades.

One potential cancer control strategy for those who are at risk of developing lung cancer is to use chemopreventive agents to inhibit the development of invasive cancer either by blocking the DNA damage that initiates carcinogenesis or by arresting or reversing the progression of premalignant cells in which such damage has already occurred (Sporn et al., 1976 ; Hong et al., 1997).

Anethole dithiolethione or 5-(p-mathoxyphenyl)-1,2-dithiole-3-thione (Sialor®, Sulfarlem®) and 5-(2-pyrazinyl)-4-methyl-1,2-dithiol-3-thione (Oltipraz) belong to the dithiolethiones chemical class of organosulfur compounds with antioxidant, chemotherapeutic, radioprotective and chemopreventive properties (Kensler et al., 1992). They represent a unique class of compounds for which anti-carcinogenic activity *in-vivo* was predicted from biochemical measurements which include induction of carcinogen detoxification enzymes. In animal carcinogenesis models, the dithiolethiones exert chemoprotective activity against development of lung as well as other cancers (Kensler et al., 1992 ; Reddy get al., 1993 ; Kensler et al., 1987 ; Bolton at al., 1993 ; Pepin et al., 1992). Thus, it has been reported that anethole trithione exerts a chemopreventive activity against colon cancer in rodent models (Reddy B. S. et al., 1993). It has also been reported that anethole dithiolethione exerts a chemopreventive effect on hamster buccal pouch carcinogenesis (Zhang L. et al., 1998). It has also been disclosed that anethole trithione exerts a chemopreventive activity on mammary cancer in rodents (Lubet Ronald A; et al., 1997). Oltipraz has been investigated in humans, but is not an approved drug. Oltipraz has been shown to protect against chemical carcinogenesis in an animal model (rodent) (Kensler et al., 1995). Recently, Oltipraz was found to be too toxic for chemoprevention (Pendyala et al., 2001). On the contrary, anethole dithiolethione (ADT) is an approved drug that has been used worldwide. In Canada, Europe and other countries, ADT is used for the treatment of drug - or radiation - induced hyposalivation, in the treatment of xerostomia from other causes (Remick et al., 1983 ; Epstein et al., 1983)), and is also marketed as a choleretic and hepatoprotective agent. At the therapeutic dose used, 25 mg three times daily, the product is safe and effective.

The present invention is based on the results of the first Phase IIb study in humans examining the potential efficacy of anethole dithiolethione (Sialor®, Sulfarlem®) in smokers with premalignant lesions in the bronchial tree.

The present invention is directed to the use of 5-(p-methoxyphenyl)-1,2-dithiole-3-thione (also called anethole dithiolethione or ADT) or a pharmaceutical derivative thereof for the preparation of a medicament for the prevention of lung cancer.

The medicament is more particularly intended for preventing or reducing the appearance of new dysplastic lesions, or the progression of pre-existing dysplastic lesions in the subject.

The medicament is advantageously also intended for improving regression of existing dysplastic lesions.

The present invention further relates to the use of 5-(p-methoxyphenyl)-1,2-dithiole-3-thione for the preparation of a medicament for preventing lung carcinogenesis in a mammalian subject having precursors of lung cancer and preferably not having cancer, especially lung cancer (*i.e.* having no history of lung cancer).

The terms "pharmaceutical derivative thereof" includes pharmaceutically acceptable salts and metabolites of 5-(p-methoxyphenyl)-1,2-dithiole-3-thione.

The terms "precursors of lung cancer" as used herein includes bronchial dysplasia, metaplasia or premalignant lesions in the bronchial tree.

"A therapeutically effective amount "as used herein refers to that amount which provides therapeutic effect for a given condition and administration regimen. Such compositions are liquids or otherwise dried formulations and include suitable diluents, preservatives, solubilizers, emulsifiers, adjuvant and/or carriers.

The composition can be administered to a subject by, for example, intraveneous, intraarterial, or intramuscular injection of a liquid preparation, oral or administration of a liquid or solid preparation. Additionally, this composition can be administered by pulmonary or nasal route.

The 5-(p-methoxyphenyl)-1,2-dithiole-3-thione or a pharmaceutical derivative thereof is preferably administered at a dosage of about 0.1 mg/kg of subject weight/day to about 50 mg/kg of subject weight/day. Preferably, the therapeutic oral dosage is of 25 mg to 100 mg once to three times daily for an adult male or female of average weight. More preferably, the therapeutic oral dosage is of 25 mg once to three times daily for an adult male or female of average weight.

The below figure and example illustrate the invention without limiting its scope.

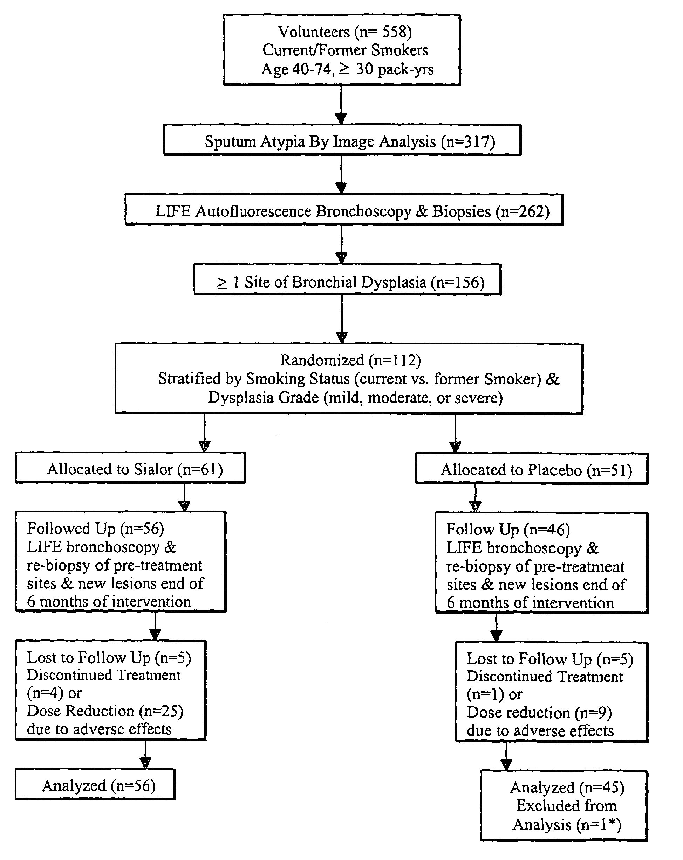

## LEGEND OF THE FIGURE:

The figure shows a flow diagram of subjects progressed through phases of the randomized trial.

One subject in the placebo group were excluded from analysis because the follow-up biopsy of the same site that showed dysplasia prior to treatment had an incomplete epithelium making it impossible to grade the response.

## EXAMPLE : A Phase II Clinical Trial of Anethole dithiolethione (Sialor®) in Smokers with Bronchial Dysplasia

In the present study, the inventors performed a randomized, double blind, placebo-controlled, Phase IIb clinical trial to determine the efficacy and safety of ADT as a chemopreventive agent in smokers with premalignant lesion of the bronchial epithelium or dysplasia.

## METHODS

One hundred and one volunteer current and former smokers with ≥30 pack-years smoking history and bronchial dysplasia identified by autofluorescence bronchoscopy directed biopsies were randomly assigned to receive ADT 25 mg orally TID or placebo for six months followed by a repeat bronchoscopy and biopsy of the same sites plus any new areas suspicious of dysplasia. Changes in the histopathology grade of the biopsies were used as the primary endpoint biomarker. All P values were two-sided.

## Clinical Trial Protocol

The flow diagram of subjects progressed through the phases of the randomized trial is shown in the annexed figure.

Assessment For Eligibility. Five hundred and fifty-eight current and former smokers over 40 years of age in the Greater Vancouver area with a smoking history of ≥30 pack-years were invited to take part in the study. A former smoker was defined as one who had given up smoking for a year or more. They were recruited through the community outreach network of the public relations department of the British Columbia Cancer Agency (BCCA) using television programs, radio broadcasts and local newspapers. Following an initial interview, which included a questionnaire to document the smoking history, a sputum sample was obtained by induction using simultaneous high frequency chest wall oscillation with the ABI vest (Advanced Respiratory Inc. St. Pauls, MN) and 3% hypertonic saline for 12 minutes. Three hundred and seventeen subjects were found to have sputum atypia by computer-assisted image analysis. Of these, 262 agreed to have an autofluorescence bronchoscopy with the LIFE-Lung device (Xillix Technologies Corp., Richmond, BC, Canada) (Lam et al., 1998 ; Lam et al., 1999). Biopsies were taken from areas with abnormal fluorescence (Lam et al., 1998; Lam et al., 1999). In addition, at least two control biopsies were taken from an upper or lower lobe. The average number of biopsies per subject was 6.7 (range 4 to 12).

One hundred and fifty-six subjects were found to have one or more sites of bronchial dysplasia on fluorescence bronchoscopy directed bronchial biopsies. One hundred and twelve subjects with bronchial dysplasia agreed to take part in the clinical trial.

Randomization. Participants with one or more sites of bronchial dysplasia were randomly assigned to receive either Sialo® (Solvay Pharma, France) at a dose of 25 mg three times daily by mouth or its placebo for six months. The placebo tablets were identical in size, shape and color to the active drug. Randomization codes were generated by a statistician at the Population and Prevention Oncology Division of BCCA and distributed to the BCCA pharmacy. All study personnel were blinded to the study coeds. Randomization was stratified according to the smoking status (current or former smoker) and dysplasia grade (mild, moderate or severe). Once a participant met the enrollment criteria, the study nurse contacted the pharmacist who then provided the study medication according to the randomization code.

Follow Up. The participants were seen monthly for examination of drug related adverse events. Liver enzymes were measured at baseline and at months 3 and 6 for toxicity monitoring. Fluorescence bronchoscopy was repeated after 6 months of study medication. The bronchoscopist was blinded to the intervention assignment. All previously biopsied sites were identified and re-biopsied under fluorescence bronchoscopy. Biopsy was also taken from new areas with abnormal fluorescence.

During the study, the current smokers were encouraged to stop smoking. They were invited to take part in the Fresh Start Program at the British Columbia Cancer Agency.

The study was approved by the Clinical Investigations Committees of the British Columbia Cancer Agency and the University of British Columbia. Informed consent was obtained from all the participants.

**Outcomes**

The primary outcome of the study was changes in the histopathology grade of the bronchial biopsies before and after six months of intervention. The secondary endpoint was changes in the nuclear morphometry index of the bronchial biopsies.

**Pathology of Bronchial Biopsies**

The biopsies were fixed in buffered formalin, embedded in paraffin, sectioned and stained with hematoxylin-eosin (H&E). Two pathologists (JCL, AFG) systematically reviewed them. The pathologists were blinded to the treatment assignments. All biopsies were classified into one of seven groups. <u>Normal</u>: as represented by pseudostratified ciliated columnar epithelium. <u>Basal cell hyperplasia</u>: as represented by an increase in the number and stratification of normal-appearing basal cells still covered with normal ciliated or mucin secreting cells. <u>Metaplasia</u>: as represented by a stratified epithelium and cytoplasmic changes consistent with squamoid differentiation. Dysplasia: mild, moderate or severe dysplasia and carcinoma *in-situ* were classified according to WHO criteria (Travis et al., 1999).

To resolve minor differences, the two pathologists consulted each other by telephone. To resolve major differences, both pathologists reviewed the slides again and, if necessary, reached consensus diagnosis after communication verbally or in person.

**Nuclear Morphometry**

The method of quantitative nuclear morphometry had been described in detail previously (Lam et al., 1998 ; Doudkine et al., 1995 ; Garner et al., 1994). McAulay et al., 1998 also suggested that high resolution quantitative morphometric measurements may be a good intermediate endpoint biomarker for chemoprevention studies of intraepithelial bronchial neoplasia. Morphometric measurements provide a more objective and reproducible means for the grading of bronchial biopsies than subjective assessment does. The device used to make these high-resolution quantitative morphometric measurements was a Cyto-Savant™ system (Lam et al., 1998 ; Doudkine et al., 1995 ; Garner et al., 1994). The images were interactively collected by an experienced cytotechnologist under the direction of a pathologist (JCL). The images were collected using a 20x objective with a numerical aperture of 0.75. Analysis of the nuclei in each tissue consisted of five steps 1) focusing the field of view, 2) automatically segmenting the nuclei in the field, 3) interactively correcting segmentation errors, 4) selecting and classifying the cell nuclei into the categories of basal, intermediate or superficial cells depending or their location within the biopsy where possible, and 5) automatically collecting individually focused images of each selected cell. Additionally, approximately 30 leukocytes were collected to normalize the images for sample to sample variations in staining intensity. On the average, greater than 100 cell nuclei were collected in this fashion for each of the biopsies. Using bulk nuclear features such as the total area and the total amount of light absorbed (which corresponds to the amount of DNA), shape features and descriptions of the distribution of stained DNA (texture features) (Doudkine et al., 1995), a scale was defined with normal nuclei defining one end and cancer-like nuclei defining the other. From the proportion of nuclei along this scale, a Morphometry Index (MI) was derived for each biopsy (Lam et al., 1998).

**Sample Size**

Information from the placebo arm of a previous trial (retinol versus placebo, NCI U01 CA68381) in this population was used to estimate the spontaneous regression rate of bronchial dysplasia. On a subject by subject analysis, the complete response rate in 38 subjects was 24%. Assuming an increase in this rate of regression of 30% (to 54%) in the ADT arm and specifying a power 0.80 for a 2-sided test at a significance level of 0.05 requires a sample size of 49 subjects per arm. It was also anticipated that each subject would present with an average of 2.4 dysplastic lesions. It was anticipated that this represented an equilibrium condition with the number of new sites being approximately equal to the number of regressing sites (data from the U01 study). The inventors modeled the appearance of new sites by a Poisson process parameter $\lambda t$, where t was elapsed time and $\lambda$ a constant, and modeled the disappearance of existing sites by a pure death process with transition rates dn = $\times$n, where n is the number of existing sites and d a constant. The resulting stochastic process, *D(t)*, describing the number of dysplastic sites had mean, $\mu$(t), where:

$$\mu(t) = \frac{\lambda}{d}[1 - e^{-dt}]$$ and variance equaled to the mean. Assuming the subjects were in an approximately stable state, the inventors approximated this by *D(t)* as *t→∞, D(∞).* This distribution was well approximated by a Poisson distribution with parameter $\lambda/d$ and could be used to estimate the sample size required to measure a change in the total number of dysplastic sites between the two arms. At the 0.05 significance level (2-sided) 50 subjects per arm will have power 0.97 to detect a 50% reduction in the number of lesions per subject (2.40 to 1.20), 0.87 to detect a 40% reduction and 0.64 to detect a 30% reduction in the number lesions.

To allow for a 10% dropout rate, it was planned that a total of 110 subjects would be randomized onto the clinical trial.

## Statistical Analyses

The primary end-point of the study was change in the histopathology grade based on the risk of progression to invasive cancer from longitudinal studies using exfoliated sputum cells and bronchial biopsies Frost et al., 1986 ; Saccomanno et al., 1982 ; Risse et al., 1988 ; Melamed et al., 1982 ; Thiberville et al., 1997 ; Shibuya et al., 2001).

For the lesion-specific analysis, complete response (CR) was defined by regression of the dysplastic lesion to hyperplasia/normal. Progressive disease (PD) was defined as appearance of lesions that were mild dysplasia or worse, irrespective of whether the site was biopsied at baseline or worsening of the dysplastic lesions present at baseline by two or more grades (e.g. mild dysplasia to severe dysplasia or worse). Partial response (PR) and stable disease (SD) referred to sites that were not CR or PD. refers to sites that are not a CR or PD. These stringent criteria to define regression or progression were based on our quantitative microscopy study that showed a significant overlap in the classification of metaplasia and mild dysplasia as well as between moderate and severe dysplasia using conventional histopathology (Lam et al., 1998).

On a participant level, response was defined as follows: CR refers to regression of all dysplastic lesions found at baseline to no higher than hyperplasia as defined by the site by site analysis at six months and no appearance of new dysplastic lesions that were mild dysplasia or worse. PD was defined as progression of one or more sites by two or more grades as defined for the lesion-specific analysis above or appearance of new dysplastic lesions that were mild dysplasia or worse at six months. Partial response (PR) was defined as regression of some but not all of the dysplastic lesions but no appearance of new lesions that were mild dysplasia or worse. Stable disease (SD) referred to subjects who did not have a CR, PR or PD.

For quantitative nuclear morphometry, the median MI for histologically normal biopsies was 1.25. The inter-observer variation of measuring the MI was such that a change in MI greater than 0.11 (>2SD) was taken to be a significant change. For the lesion specific analysis, CR was defined as regression of a lesion with a MI of $> 1.36$ to $\leq 1.36$. PD was defined as increase in the MI from $\leq 1.36$ to $> 1.36$. On a participant by participant basis, CR was defined as regression of all sites with MI $> 1.36$ to $\leq 1.36$. PD was defined as progression of any site similar to that defined under the site by site analysis or appearance of new lesions with a MI of $> 1.36$.

Descriptive statistics were used to summarize subject characteristics, and pathologic evaluations of the bronchial biopsy examinations. Comparison between treatment arms was done with the Mann-Whitney test for continuous variables such as age, smoking intensity (pack-years) and MI. Pearson's $\chi^2$ test with continuity correction was used to compare categorical variables such as gender, smoking status (current versus former smokers) and response rates (progression and regression) in the two arms. Fishers exact test was applied to the lower partial response and stable disease rates. All P values are two-sided. A two-sided P value of less than .05 was considered statistically significant.

To adjust for the effect of various pre-treatment factors on the likelihood of regression or progression of dysplastic lesions, a multiple logistic regression analysis was used on a participant level. This analysis included the following variables: age, sex, smoking status and the smoking intensity (pack-years). All analyses were unconditional, and tests of statistical significance and confidence intervals (CIs) for odds ratios (ORs) were based on the log-likelihood test.

## RESULTS

Summary of the results : In the lesion specific analysis, progression of pre-existing dysplastic lesions by two or more grades and/or appearance of new lesion was 9% lower in the ADT group (8% ADT versus 17% placebo, P<0.001). In the person specific analysis, the progression rate was 22% lower (32% ADT versus 59% placebo, P=0.01). Nuclear morphometry of the biopsies was used as a secondary endpoint. In half of the participants who had elevated morphometry indices pre-treatment, the progression rate was 19% lower (41% ADT versus 60% placebo, P=0.28). Adverse events were mostly minor gastrointestinal symptoms that resolved with dose reduction or discontinuation of the medication.

Detailed results :

Of the 112 subjects that were randomly assigned to ADT or placebo, 61 were allocated to the ADT arm and 51 to the placebo arm (Figure). One hundred and two subjects completed the six months study. Ten subjects dropped out unrelated to side effects of the study medication. They did not return for the six months follow-up bronchoscopy and hence were excluded from the analyses since changes of the histopathology grades of the bronchial biopsies were used as the primary endpoint to assess the efficacy of the chemopreventive treatment An additional subject in the placebo group was excluded from the analysis because the follow-up biopsy of the same site that showed dysplasia prior to treatment had incomplete epithelium for making a pathological diagnosis. The remaining 101 subjects who had taken one or more doses of the study medication and had a follow-up bronchoscopy after six months of intervention were included in the analysis.

The characteristics of the 101 participants who completed the trial are shown in Table 1. There was no significant difference in the age, sex and smoking history between the ADT and placebo groups. There were fewer former smokers in the placebo arm (18% versus 34% in the ADT group) but the difference was not significant (p=0.07).

Table 1 : Characteristics of Participants in this Study

| | | ADT | Placebo |
|---|---|---|---|
| Number of Participants | | 56 | 45 |
| Age (Years) | Median | 54 | 54 |
| | Range | 41-74 | 43-71 |
| Gender | Male | 36 (64%) | 26 (58%) |
| | Female | 20 (36%) | 19 (42%) |
| Smoking | Median | 48 | 47 |
| (Pack-Years) | Range | 30-99 | 32-172 |
| | Current Smoker | 37(66%) | 37(82%)* |
| | Former Smoker | 19(34%) | 8(18%) |
| Highest Grade Dysplasia | Mild | 44 (79%) | 37 (82%) |
| | Moderate/Severe | 12 (22%) | 8 (18%) |
| Number With ≥ 2 Sites of Dysplasia | | 37 (66%) | 29 (64%) |

\* P=0.07 compared to Anethole dithiolethione (ADT) group by chi-square test.

**Effects of Sialor on Histopathology**

Lesion Specific Analysis

At six months, the complete response rate was 12% higher in the ADT group than the placebo group (53% versus 41%, p=0.14, not significant). The progression rate was significantly lower in the ADT group than the placebo group (8% versus 17%, p<0.001) (Table 2A).

Table 2 : Pathology Grades of Bronchial Biopsies Before And Six Months After Intervention: Lesion Specific and Person Specific Analyses.

**A. Lesion Specific Analysis**

| Pathology Grade of Bronchial Biopsies | | 6 Months Follow-up Bronchial Biopsies | | | | | |
|---|---|---|---|---|---|---|---|
| | | 1 | 2 | 3 | 4 | 5 | Total |
| Baseline Placebo | Not sampled+ | 17 | 12 | **19** | **4** | 0 | 52 |
| | 1 | 82 | **4** | **14** | 0 | 0 | 100 |
| | 2 | 10 | 2 | **8** | 0 | 0 | 20 |
| | 3 | **36** | 13 | 33 | 1 | 1 | 84 |
| | 4 | 3 | 3 | 3 | 1 | 0 | 10 |
| | 5 | 0 | | 0 | 0 | 0 | 0 |
| | Total | 148 | 34 | 77 | 6 | 1 | 266 |
| Baseline ADT | Not sampled | 34 | 8 | **13** | 0 | 1 | 56 |
| | 1 | 100 | 9 | **5** | **2** | 0 | 116 |
| | 2 | 22 | 2 | **3** | 0 | 0 | 27 |
| | 3 | **53** | 14 | 27 | 6 | **0** | 100 |
| | **4** | **8** | 0 | 2 | 3 | 1 | 14 |
| | 5 | 0 | 0 | 2 | 0 | 0 | 2 |
| | Total | 217 | 33 | 52 | 11 | 2 | 315 |

*1= normal/hyperplasia, 2= metaplasia, 3= mild dysplasia, 4= moderate dysplasia, 5= severe dysplasia.
+Not sampled refers to additional biopsies taken at the six months follow-up bronchoscopy.
Bold areas on the left represent regression (CR), only dysplasia sites could regress to normal/hyperplasia. Bold areas on the right represent progression (PD). Any site at baseline could progress. New dysplastic lesions not sampled at baseline were considered as PD as the enrollment criteria was bronchial dysplasia. CR was 53% (61/116) and 41% (39/94) in the ADT and placebo groups respectively, P=0.14, $\chi^2$ test). PD was 8% (24/315) and 17% (46/266) in the ADT and placebo groups respectively, P<0.001, $\chi^2$ test). All P-values are two-sided.

**B. Person Specific Analysis**

| | CR | PR | SD | PD | Excluded | Total |
|---|---|---|---|---|---|---|
| Placebo | 7 (16%) | 7 (16%) | 4 (9%) | 26 (54%) | 1+ | 44 |
| ADT | 15 (27%) | 13 (23%) | 10 (18%) | 18 (32%) | | 56 |
| p-values* | 0.29 | NA | NA | 0.013 | | |

* Comparison of proportions with chi-square test. All P values are two-sided. NA : not applicable
+One Placebo participant whose baseline dysplasia sites could not be scored at 6 months because of incomplete epithelium was omitted from the comparison.

Person Specific Analysis

The complete response rate was 11% higher in the ADT group versus placebo, (27% versus 16%, p=0.29). The PD rate was 22% lower for the ADT group versus the placebo group (32% versus 54%). The difference was statistically significant, P=0.013, $\chi^2$ test)(Table 2B). Seven of the 37 current smokers (19%) in the ADT group and 6/37 current smokers (16%) in the placebo group gave up smoking during the study. Eleven of the 37 current smokers in the ADT group increased their smoking during the study and 15 decreased their smoking. The corresponding figures for the placebo group were 6/37 and 21/37, respectively. The average percentage change in smoking compared to baseline was 34% in the ADT group and 37% in the placebo group.

Multiple logistic regression analysis was used to determine simultaneous effect of age, gender, smoking history and effect of treatment on progression (PD) (Table 3).

Table 3 : Likelihood Of Progressive Disease Six Months After Intervention

| Significant factors: | | OR* | 95% Confidence Interval | P-value |
|---|---|---|---|---|
| Treatment | Placebo | 3.5 | 1.3 - 9.3 | 0.01 |
| | ADT | 1 | | |
| Gender | Men | 6.2 | 2.1 -17.8 | 0.001 |
| | Women | 1 | | |
| Smoking status | Current-smoker | 3.4 | 1.1 - 10.8 | 0.03 |
| | Former smoker | 1 | | |
| Cigarettes Smoked (per 1 pack-year) | | 1.0 | 1.0 - 1.1 | 0.07 |
| *Multiple regression analysis | | | | |

Placebo subjects were estimated to have 3.5 times higher odds of progression (95% CI = 1.3 - 9.3, p=0.013). Current smokers had 3 time higher odds of PD than ex-smokers (95% CI = 1.1 - 10.8, p=0.03). Gender was estimated to have the strongest effect on PD with men having 6 time higher odds of PD than women (95% CI = 2.1 -17.8, p=0.001).

Multiple logistic regression analysis was also used to determine simultaneous effect of age, gender, smoking history and effect of treatment on regression (CR). ADT treatment was not significantly associated with increasing odds of CR. Smoking as measured by pack-years decreased the odds of CR by 10% for each additional 1 pack-year of smoking (OR 0.9, 95% CI = 0.84 - 0.96, p=0.002). This means that a person with 10 pack-year heavier smoking was estimated to have 64% lower odds of CR than another subject with similar age and gender but less smoking. For a person 10 years older at baseline (given the same smoking intensity and gender), the model estimated 3.3 fold higher odds of CR (95% CI = 1.6 - 7.1, p=0.002). Gender had a borderline significant association with CR suggesting that females had on average 3 times higher odds of CR (95% CI 0.96 - 9.3, p=0.06).

**Effect of Dose Reduction on Histopathology**

Thirty-one subjects were able to take the full 25 mg TID dose of ADT for 6 months. The dose was reduced to 25 mg BID in 17 subjects, 25 mg QD in 4 subjects and discontinued in 4 subjects. There was no significant difference in the CR or PD rates (all P-values >0.90) between those who were on the full dose compared to those on a reduced dose (Table 4).

Table 4. Effect of Dose Reduction on Response Rates

| Dose Reduction | CR | PR | SD | PD | Total |
|---|---|---|---|---|---|
| No | 9 (29%) | 5 (16%) | 7 (23%) | 10 (32%) | 31 |
| Yes | 6 (24%) | 8 (32%) | 3 (12%) | 8 (32%) | 25 |
| Total | 15 | 13 | 10 | 18 | 56 |

(continued)

| Dose Reduction | CR | PR | SD | PD | Total |
|---|---|---|---|---|---|
| P-value* | 0.91 | NA | | 0.98 | |
| * Chi-square test<br>NA : not applicable | | | | | |

### Effect of ADT on Nuclear Morphometry

Fifty-two percent of the subjects in the ADT group and 57% of the subjects in the placebo group had at least one biopsy that had a MI >1.36. In the person specific analysis, the CR rate was 21% higher in the ADT group than in the placebo (45% versus 24%, p=0.19). The PD rate was 19% lower (41% versus 60%, p=0.28)(Table 5). Table 5 : Nuclear Morphometry of Bronchial Biopsies Before and Six Months after Intervention: Lesion Specific and Person Specific Analyses

A. Lesion Specific Analysis

| Baseline Biopsies | 6 months | | | |
|---|---|---|---|---|
| | MI | ≤1.36 | >1.36 | total |
| Placebo | ≤1.36 | 66 | 32 (33%) | 98 (100%) |
| | >1.36 | 19 (63%) | 11 | 30 (100%) |
| | Total | 85 | 43 | 128 |
| ADT | ≤1.36 | 85 | 27 (24%) | 112 (100%) |
| | >1.36 | 29 (76%) | 9 | 38 (100%) |
| | Total | 114 | 36 | 150 |
| Comparison of regression rates between ADT and Placebo p=0.37, chi-square test<br>Comparison of progression rates p=0.22, chi-square test | | | | |

B. Subject Specific Analysis

| | CR | PR | SD | PD | Total |
|---|---|---|---|---|---|
| Placebo | 6 (24%) | 1 (4%) | 3 (12%) | 15 (60%) | 25 (100%) |
| ADT | 13 (45%) | 1 (3%) | 3 (10%) | 12 (41%) | 29 (100%) |
| p-values | 0.19* | NA | | 0.28* | |
| * Comparison using chi-square test for equality of proportions<br>NA : not applicable | | | | | |

In the lesion specific analysis, the CR rate was 76% in the ADT group and 63% in the placebo group. The corresponding PD rates were 24% and 33% respectively. The difference between the two groups was not significant (P=0.37 and 0.22 for CR and PD, respectively). (Table 5).

### Adverse Events

Symptoms of excessive flatus, abdominal bloating, loose stool and constipation were frequently reported by participants in both groups (Table 6).

Table 6. Adverse Events

| Symptom | Placebo | ADT |
|---|---|---|
| Excessive Flatus | 53% | 93% |

(continued)

| Symptom | Placebo | ADT |
|---|---|---|
| Abdominal Bloating | 4% | 25%* |
| Loose Stool | 8% | 16% |
| Diarrhea | 12% | 13% |
| Constipation | 0% | 5% |
| Increased Saliva | 4% | 2% |
| * P= 0.01 compared to placebo (chi-square test) | | |

Only abdominal bloating was significantly more frequent in the ADT group (P=0.018). Grade 2 symptoms were observed in 51% of the participants taking ADT versus 20% of those on placebo. Grade 3 symptoms were observed in 11% of those taking ADT and 6% of those on placebo. One of the participants taking ADT had a Grade 3 elevation of liver enzymes. The liver enzymes returned to normal after discontinuation of the ADT. Dose reduction was required in 45% of the participants taking ADT and 25% of those on placebo. The study medication had to be discontinued in four of the subjects in the ADT group and one of the subjects in the placebo group because of complaints of minor, though intolerable gastrointestinal symptoms.

**DISCUSSION**

In the primary endpoint analyses, a significantly lower rate of progression of pre-existing dysplastic lesions by two or more grades and/or appearance of new lesions were observed after 6 months of ADT at a dose of 25 mg orally TID compared to the placebo.

At the present time, bronchial dysplasia is one of the best surrogate endpoint biomarker to assess the effect of new chemopreventive agents. The morphological criteria for pre-invasive lesions have been defined in the recent WHO classification (Travis et al., 1999). Grading of squamous pre-invasive lesions was found to be reproducible. In humans, the presence of dysplastic cells in sputum cytology or bronchial biopsy is associated with the development of invasive lung cancer in prospective studies (Frost et al., 1986 ; Saccomano et al., 1982 ; Risse et al., 1988 ; Melamed et al., 1982 ; Thiberville et al., 1997 ; Shibuya et al., 2001) similar to what is known from cancer progression models in animals (Nasiell et al., 1987). Reversal of dysplasia with successful modulation is associated with reduced cancer risk (Boone et al., 1992 ; Boone et al., 1997). To minimize inter-observer variation, dysplastic changes in the bronchial epithelium can be quantitated by image cytometry (Lam et al., 1998 ; Boone et al., 1992 ; Boone et al., 1997) as is done in the current study. Using quantitative nuclear morphometry we observed a 21% better CR rate and a 19% lower PD rate in the ADT group versus placebo. Although the differences were not statistically significant, the magnitude of the difference between the two groups was similar to that observed using histopathology criteria (Travis et al., 1999). A greater statistical significance might have been observed if both histopathology and nuclear morphometry were used as the enrollment criteria as only 50% of our subjects had at least one biopsy with MI >1.36 at baseline. The inventors did not use nuclear morphometry in the inclusion criteria in this clinical trial because it was developed after the study began.

This is the first Phase II lung cancer chemoprevention trial using bronchial dysplasia as the primary intermediate endpoint biomarker. Previous studies used the metaplasia index or a combination of metaplasia and dysplasia with very few dysplastic lesions present in the participants (Lee et al., 1994 ; Kurie et al., 2000). Using metaplasia as the intermediate endpoint biomarker, isotretinoin and N-(4-Hydroxyphenyl)retinamide were not found to be effective chemopreventive agents (Lee et al., 1994 ; Kurie et al., 2000). The effect of these agents on bronchial dysplasia is not known. Similar to these studies of retinoids (Lee et al., 1994 ; Lippman et al., 2001), in addition to ADT effect, the inventors observed a difference between former and current smokers in the development and progression of dysplastic lesions. The reason why women had better response than men is not known and requires further investigation.

The mechanisms of action of ADT are multiple. It exerts effects on glutathione. After administration of ADT, an increase of intracellular glutathione has been shown in animals pretreated with various toxins as well as in normal animals. This effect is produced by a stimulation of glutathione synthesis via the glutamyl cysteine synthetase and is accompanied by an increase of glutathione-dependent enzyme activity (Wamet et al., 1989). Increase of glutathione-S-transferase activity, a phase II enzyme implicated in chemoprevention against aflatoxin hepatocarcinogenesis, induced by ADT, can explain its protective action against aflatoxin tumorigenicity observed in rats by Kensler et al., 1987. It is also a potent inhibitor of lipid peroxidation, as demonstrated *in-vitro* in a rat liver microsome model. It exerts free radical scavenger properties evidenced among others by radiolysis studies performed in various conditions (Christen et al.,

1995 ; Christen et al., 1996), and thereby it protects the cellular membrane by inhibiting lipid peroxidation and diene formation. Moreover, ADT was observed to be capable of modulating the nuclear factor NFκB (a redox sensitive cytolytic transcription factor) activation in human Jurkat cells (Sen et al., 1996). Pretreatment of human Jurkat cells by ADT significantly protects cells against oxidative stress-induced cytotoxicity (Khanna et al., 1998). Although dithiolethiones may act via several mechanisms including inhibition of cell replication, it appears that they act predominantly by carcinogen detoxification via up-regulation of phase II enzymes including glutathione-S-transferase (GST). In a chemoprevention setting using a rat model of hepatoma, the mean GST levels in the liver were statistically increased (p<0.01) both by ADT and Oltipraz. The GST level was found to be elevated by 3.2 to 4.5-fold after changing the concentration of Oltipraz in the diet from 0.01% to 0.1% (Kensler et al., 1987). In the human, a study in Qidong, China showed that intermittent, high-dose Oltipraz inhibited phase I activation of aflatoxins, and sustained low-dose Oltipraz increased phase II conjugation of aflatoxin (Wang et al., 1999). The detoxification and anti-oxidant actions may explain the greater effect of ADT in preventing appearance of new dysplastic lesions or progression of existing dysplastic lesions to a higher grade than regression of existing dysplastic lesions observed in this study. Administration of ADT longer than 6 months in a larger number of participants may show the effect of ADT on regression of existing dysplastic lesions better.

In this Phase II study, the inventors used the dosage of ADT that was approved by Health Canada for the treatment of xerostomia in order to establish the potential efficacy of this agent in smokers with bronchial dysplasia. The only adverse events observed were gastrointestinal. This is in keeping with previous clinical data on the safety profile of ADT in the treatment of patients with dry mouth (Remick et al., 1983). Since the activity of the drug was observed despite 45% of the participants were taking the medication only twice or once a day, the possibility that effective chemopreventive activity can be achieved in a two times a day or once daily dosage needs to be investigated further.

This study showed for the first time in smokers with pre-malignant lesions that ADT (Sialor®, Sulfarlem®) statistically reduces the appearances of new dysplastic lesion or progression of pre-existing dysplastic lesions. Given that more than half of all long-term smokers are unable to stop smoking despite aggressive behavioral and pharmacologic smoking cessation measures (Wang et al., 1999), this study suggests new strategies for lung cancer control using chemoprevention by the free radical scavenger and glutathione inducer anethole dithiolethione (Sialor®, Sulfarlem®).

## REFERENCES

- Bolton MG, Munoz A, Jacobson LP, Groopman JD, Maxuitenko YY, Roebuck BD, et al. Transient intervention with Oltipraz protects against aflatoxin-induced hepatic tumorigenesis. Cancer Res 1993; 53:3499-504.
- Boone CW, Bacus JW, Bacus JV, Steele VE, Kelloff GJ. Properties of intraepithelial neoplasia relevant to the development of cancer chemopreventive agents. J Cell Biochem, Suppl 1997;28/29:1-20.
- Boone CW, Kelloff GJ, Steele VE. Natural history of intraepithelial neoplasia in humans with implications for cancer chemoprevention strategy. Cancer Res 1992;52:1651-1659.
- Christen MO. Anethole Dithiolethione : Research overview and perspectives. In: Packer L, Traber MG, Xin W, editors. Molecular Mechanisms and Health Effects. AOCS Press; 1996. p. 236-242.
- Christen MO. Anethole dithiolethione: Biochemical considerations. Methods in Enzymology 1995;252:316-323.
- Doudkine A, MacAulay C, Poulin N, Palcic B. Nuclear texture measurements in image cytometry. Pathologica 1995; 87(3):286-289.
- Epstein JB, Decoteau WE, Wilkinson A. Effect of Sialor in treatment of xerostomia in Sjogren's syndrome. Oral Surg Oral Med Oral Pathol 1983;56:495-9.
- Frost JK, Ball WC Jr, Levin MI, Tockman MS, Erozan YS, Gupta PK, et al. Sputum cytology: use and potential in monitoring the workplace environment by screening for biological effects of exposure. J Occup Med 1986;28:692-703.
- Garner DM, Harrison A, MacAulay C, Palcic B. Cyto-Savant™ and its use in automated screening of cervical smears. In: Wied GL, Bartels PH, Rosenthal PH, Schenck U, editors. Compendium on the Computerized Cytology and Histology Laboratory. Chicago: Tutorials of Cytology; 1994. p. 346-352.
- Greenlee RT, Hill-Harmon MB, Murray T, Thun M. Cancer Statistics, 2001. CA Cancer J Clin 2001;51:15-36.
- Halpern MT, Gillespie BW, Warner KE. Patterns of absolute risk of lung cancer mortality in former smokers. J Natl Cancer Inst 1993;85:457-464.
- Hong WK, Spom MB. Recent advances in chemoprevention of cancer. Science 1997;278:1073-1077.
- Jorenby DE, Lelschow S, Nides M, Rennard S, et al. A controlled trial of sustained-release bupropion, a nicotine patch or both for smoking cessation. N Engl J Med 1999;340:681-91.
- Kensler TW, Egner PA, Dolan PM, Groopman JD, Roebuck BD. Mechanism of protection against aflatoxin tumorigenicity in rats fed 5-(2-pyrazlnyl)-4-methyl-1,2-dithiol-3-thione (oltipraz) and related 1,2-dithiol-3-thiones and 1.2-dithlol-3-ones. Cancer Res 1987;47:4271-7.
- Kensler TW, Groopman JD, Roebuck BD. Chemoprevention by Oltipraz and other ditholethiones. In: Wattenberg

- L, Lipkin M, Boone C, Kelloff G, editors. Cancer Chemoprevention. Boca Raton; CRC Press; 1992. p. 205-225
- Kensler T.W. et al., "Oltipraz, Clinical Opportunities for Cancer Chemoprevention", Journal of Cellular Biochemistry, Supplement, Wiley-Liss, US, vol. 22, 1995, p. 101-107.
- Khanna S, Sen CK, Roy S, Christen MO, Packer L. Protective effects of anethol dithiolthione against oxidative stress induced cytotoxicity in human Jurkat T cells. Biochem Pharmacol, 1998;56:61-69.
- Kurie JM, Lee JS, Khuri FR, et al. N-(4-Hydroxyphenyl)retinamide in the chemoprevention of squamous metaplasia and dysplasia of the bronchial epithelium. Clin Cancer Res, 2000;6:2973-9.
- Lam S, Kennedy T, Unger M, Miller Y, Gelmont D, Rusch V, et al. Localization of bronchial intraepithelial neoplastic lesions by fluorescence bronchoscopy. Chest 1998;113:696-702.
- Lam S, leRiche JC, Zheng Y, Coldman A, MacAulay C, Hawk E, et al. Sex-related differences In bronchial epithelial changes associated with tobacco smoking. J Natl Cancer Inst 1999;91:691-696.
- Lam S, MacAulay CE. Endoscopic localization of preneoplastic lung lesions. In: Martinet Y, Hirsch FR, Martinet N, Vignaud JM, Mulshine JL, editors. Clinical and Biological Basis of Lung Cancer Prevention. Switzerland; Birkhauser Verlag Basel; 1998, p.231-237.
- Lee JS, Lippman SM, Benner SE, Lee JJ, Ro JY, Lukeman JM, et al. Randomized placebo-controlled trial of isotretinoin in chemoprevention of bronchial squamous metaplasia, J Clin Oncol, 1994;12:937-945.
- Lippman SM, Lee JJ, Karp DD, Vokes EE, Benner SE, Goodman GE, et al. Randomized phase III intergroup trial of isotretinon to prevent second primary tumors in stage I non-small cell lung cancer. J Natl Cancer Inst 2001;93: 605-18.
- Lubet Ronald, A. et al., "Chemopreventive efficacy of anethole trithione, N-acetyl-L-cysteine, miconazole and phenethylisothiocyanate in the DMBA-induced rat mammary cancer model", International Journal of Cancer, vol. 72, n° 1, 1997,
- Mac Aulay CE, Lam S, Klein-Parker H, Gadzar A, Guillaud M, Payne P, le Riche J, Dawe C, Band P, and Palcic B. Intermediate endpoint biomarkers for lung cancer chemoprevention, "PROC.SPIE - Int, Soc, Opt. Eng. 1998; 3260: 207-210.
- Melamed MR, Zaman MB. Pathogenesis of epidermoid carcinoma of lung. In: Shimosato Y, Melamed MR, Nettesheim P, editors. Morphogenesis of lung cancer, Vol I. Boca Raton: CRC Press, 1982. p 37-64.
- Nasiell M, Auer G, Kato H. Cytological studies in man and animals on development of bronchogenic carcinoma. In: McDowell EM, editor. Lung Carcinomas. Edinburgh: Churchill Livingstone; 1987, p. 207-242.
- Pendyala L, Schwartz G, Bolanowska-Higdon W, Hitt S, Zdanowicz J, Murphy M, et al. Phase I/phan-nacodynamic study of N-acetyicysteine/Otipraz in smokers: early termination due to excessive toxicity. Cancer Epidemiol Biomarkers Prev 2001;10:269-72.
- Pepin P, Bouchard L, Nicole P, Castoguay A. Effects of sulindac and Oltipraz on the tumorigenicity of 4-(methynitrosamino)1-(3-pyridyl)-1-butanone in A/J mouse lung. Carcinogenesis 1992;13:341-348.
- Reddy BS, Rao CV, Rivenson A, Kelloff G. Chemoprevention of colon carcinogenesis by organosulfur compounds. Cancer Res 1993;53:3493-8.
- Remick RA, Blasberg B, Patterson BD, Carmichael RP, Miles JE. Clinical aspects of xerostomia. J Clin Psychiatry 1983;44:63-5.
- Risse EKJ, Vooijs GP, van't Hof MA. Diagnostic significance of "severe dysplasia" in sputum cytology, Acta Cytologica 1988;32:629-634.
- Saccomanno G. Carcinoma in situ of the lung: Its development, detection, and treatment. Semin Respir Med 1982; 4(2):156-60.
- Sen CK, Traber KE, Packer L. Inhibition of NF-$\kappa$B Activation in Human T-Cell Lines by Anetholdithiolthione. Biochem Biophys Res Commun 1996;218:148-153.
- Shibuya K, Fujisawa T, Hoshino H: A follow up study of squamous dysplasia. J Clin Exp Med 2001;199:593-596.
- Spom MB, Dunlop NM, Newton DL, Smith JM. Prevention of chemical carcinogenesis by Vitamin A and its synthetic analogs. Fed Proc 1976;35:1332-1338.
- Thiberville L, Metayer J, Raspaud C, Nouvet G. A prospective, short term follow-up study of 59 severe dysplasias and carcinoma in-situ of the bronchus using autofluorescence endoscopy. Eur Respir J 1997;10:425S.
- Tong L, Spitz MR, Fueger JJ, Amos CA. Lung cancer in former smokers. Cancer 1996; 78:1004-1010.
- Travis WD, Colby TV, Corrin B, Shimosato Y Brambilla E. Histologic and graphical text slides for the histological typing of lung and pleural tumors. In: World Health Organization Pathology Panel: World Health Organization. International Histological Classification of Tumors. 3rd ed. Berlin: Springer Verlag; 1999. p 5.
- Wang JS, Shen X, He X, Zhu YR, Zhang BC, Wang JB, et al. Protective alterations in phase 1 and 2 metabolism of aflatoxin B1 by Oltipraz in residents of Qidong, People's Republic of China. J Natl Cancer Inst 1999;91(4);347-54.
- Warnet JM, Christen MO, Thevenin M, Biard D, Jacqueson A, Claude JR, Protective effect of anethol dithiolthione against acetaminophen hepatotoxicity in mice. Pharmacol Toxicol 1989;65:63-64.
- Zhang L. et al., "The chemopreventive effect of Sialor (Anethole dithiolethione) on hamster buccal pouch carcinogenesis", Proceedings of the American Association for Cancer Research Annual, vol. 39, March 1998, p. 641.

**Claims**

1. Use of 5-(p-methoxyphenyl)-1,2-dithiole-3-thione or of a pharmaceutical derivative thereof for the preparation of a medicament for preventing lung cancer in a mammalian subject.

2. The use of claim 1, wherein the subject has precursors of lung cancer.

3. The use of claim 2, wherein the subject has bronchial dysplasia, metaplasia, or premalignant lesions in the bronchial tree.

4. The use of any of claims 1 to 3, wherein the subject has no history of lung cancer.

5. The use of any of claims 1 to 4, wherein the medicament is intended for preventing or reducing the appearance of new dysplastic lesions, or the progression of pre-existing dysplastic lesions in the subject.

6. The use of any of claims 1 to 4, wherein the medicament is intended for improving regression of existing dysplastic lesions.

7. The use of any of claims 1 to 6, wherein the pharmaceutical derivative is a pharmaceutically acceptable salt of 5-(p-methoxyphenyl-1,2-dithiole-3-thione.

8. The use of any of claims 1 to 6, wherein the pharmaceutical derivative is a pharmaceutically acceptable metabolite of 5-(p-methoxyphenyl)-1,2-clithiole-3-thione.

**Patentansprüche**

1. Verwendung von 5-(p-Methoxyphenyl)-1,2-dithiol-3-thion oder eines pharmazeutischen Derivats davon für die Herstellung eines Arzneimittels zur Vorbeugung von Lungenkrebs bei einem Säuger-Patienten.

2. Verwendung nach Anspruch 1, worin der Patient Lungenkrebs-Vorläufer aufweist.

3. Verwendung nach Anspruch 2, worin der Patient an Bronchialdysplasie, Metaplasie oder prämalignen Läsionen im Bronchialtrakt leidet.

4. Verwendung nach einem der Ansprüche 1 bis 3, worin der Patient keine Lungenkrebs-Historie aufweist.

5. Verwendung nach einem der Ansprüche 1 bis 4, worin das Arzneimittel zur Vorbeugung oder Reduktion des Auftretens von neuen-dysplastischen Läsionen oder zur Vermehrung vorexistierender dysplastischer Läsionen des Patienten bestimmt ist.

6. Verwendung nach einem der Ansprüche 1 bis 4, worin das Arzneimittel zur Verbesserung der Regression von existierenden dysplastischen Läsionen bestimmt ist.

7. Verwendung nach einem der Ansprüche 1 bis 6, worin das pharmazeutische Derivat ein pharmazeutisch annehmbares Salz von 5-(p-Methoxyphenyl)-1,2-dithiol-3-thion ist.

8. Verwendung nach einem der Ansprüche 1 bis 6, worin das pharmazeutische Derivat ein pharmazeutisch annehmbarer Metabolit von 5-(p-Methoxyphenyl)-1,2-dithiol-3-thion ist.

**Revendications**

1. Utilisation de 5-(p-méthoxyphényl)-1,2-dithiole-3-thione ou d'un dérivé pharmaceutique de celui-ci, pour la préparation d'un médicament destiné à éviter le cancer du poumon chez un sujet mammifère.

**2.** Utilisation selon la revendication 1, dans laquelle le sujet a des précurseurs de cancer du poumon.

**3.** Utilisation selon la revendication 2, dans laquelle le sujet a une dysplasie bronchique, une métaplasie, ou des lésions pré-malignes dans l'arbre bronchique.

**4.** Utilisation selon l'une quelconque des revendications 1 à 3, dans laquelle le sujet n'a aucune histoire de cancer du poumon.

**5.** Utilisation selon l'une quelconque des revendications 1 à 4, dans laquelle le médicament est destiné à éviter ou réduire l'apparition de nouvelles lésions dysplasiques, ou la progression de lésions dysplasiques existantes chez le sujet.

**6.** Utilisation selon l'une quelconque des revendications 1 à 4, dans laquelle le médicament est destiné à améliorer la régression de lésions dysplasiques existantes.

**7.** Utilisation selon l'une quelconque des revendications 1 à 6, dans laquelle le dérivé pharmaceutique est un sel pharmaceutiquement acceptable de 5-(p-méthoxyphényl)-1,2-dithiole-3-thione.

**8.** Utilisation selon l'une quelconque des revendications 1 à 6, dans laquelle le dérivé pharmaceutique est un métabolite pharmaceutiquement acceptable de 5-(p-méthoxyphényl)-1,2-dithiole-3-thione.

Volunteers (n= 558)
Current/Former Smokers
Age 40-74, ≥ 30 pack-yrs

Sputum Atypia By Image Analysis (n=317)

LIFE Autofluorescence Bronchoscopy & Biopsies (n=262)

≥ 1 Site of Bronchial Dysplasia (n=156)

Randomized (n=112)
Stratified by Smoking Status (current vs. former Smoker) &
Dysplasia Grade (mild, moderate, or severe)

Allocated to Sialor (n=61)

Allocated to Placebo (n=51)

Followed Up (n=56)
LIFE bronchoscopy &
re-biopsy of pre-treatment
sites & new lesions end of
6 months of intervention

Follow Up (n=46)
LIFE bronchoscopy &
re-biopsy of pre-treatment
sites & new lesions end of
6 months of intervention

Lost to Follow Up (n=5)
Discontinued Treatment
(n=4) or
Dose Reduction (n=25)
due to adverse effects

Lost to Follow Up (n=5)
Discontinued Treatment
(n=1) or
Dose reduction (n=9)
due to adverse effects

Analyzed (n=56)

Analyzed (n=45)
Excluded from
Analysis (n=1*)